Europäisches Patentamt

European Patent Office

Office européen des brevets

(19)

(11) Veröffentlichungsnummer: **0 221 273 B1**

# (12) EUROPÄISCHE PATENTSCHRIFT

(45) Veröffentlichungstag der Patentschrift: **17.04.91**

(51) Int. Cl.<sup>5</sup>: **A61F 2/32**

(21) Anmeldenummer: **86111845.3**

(22) Anmeldetag: **27.08.86**

(54) **Schaft für eine Femurkopfprothese.**

(30) Priorität: **03.10.85 CH 4278/85**

(43) Veröffentlichungstag der Anmeldung:
**13.05.87 Patentblatt 87/20**

(45) Bekanntmachung des Hinweises auf die
Patenterteilung:
**17.04.91 Patentblatt 91/16**

(84) Benannte Vertragsstaaten:
**AT DE FR GB IT**

(56) Entgegenhaltungen:
**DE-A- 2 320 683**
**DE-A- 2 322 101**
**FR-A- 1 278 359**
**US-A- 3 939 498**
**US-A- 4 404 692**

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**

**Beschreibung**

Die Erfindung betrifft einen kragenlosen Schaft für eine Femurkopfprothese, der medial in einem Bogen in den Prothesenhals übergeht und im eingesteckten Zustand in seinem distalen Bereich fest im Knochen verkeilt ist, wobei im proximalen Bereich des medialen Bogens für die Aufnahme einer Schraube eine Bohrung vorgesehen ist, die den Schaft als Durchgangsbohrung durchsetzt und mindestens annähernd normal durch die Oberfläche des medialen Bogens verläuft, sowie eine Auflagefläche für den Kopf einer Knochenschraube enthält.

Ein Schaft der vorstehend genannten Art ist beispielsweise aus der DE-A-2 322 101 bekannt. Dieser bekannte Schaft weist eine Gewindebohrung auf, die ihn von lateral nach medial durchsetzt. In diese Bohrung wird eine Schraube von lateral her eingeschraubt, um den grossen Trochanter an der Prothese zu halten, ihn beispielsweise zwischen Schraubenkopf und Implantat einzuklemmen.

Bei Schäften, die distal fest im Knochen verkeilt sind, hat sich gezeigt, dass es zu Mikrobewegungen im Bereich des Calcarbogens kommt, die zu einem Knochenabbau führen und schliesslich eine Lockerung der Prothese verursachen. Diese Mikrobewegungen sind durch pulsierende Druckkräfte verursacht, die bei "eingespanntem" distalen Ende aufgrund der Elastizität des Schaftes infolge der Biegemomente durch Be- und Entlastungen des Gelenkkopfes auftreten. Bei der zementfreien Verankerung tritt zusätzlich das Problem der Primärfixation im proximalen Schaftbereich auf.

Aufgabe der Erfindung ist es, bei einer kragenlosen, vorwiegend zementfrei verankerbaren Femurkopfprothese, die in ihrem distalen Bereich fixiert wird, die geschilderten Mikrobewegungen am Calcarbogen zu verhindern. Diese Aufgabe wird mit der Erfindung dadurch gelöst, dass die Bohrung für die Aufnahme der Knochenschraube gewindefrei ausgebildet ist.

Mit Hilfe einer Schraube, die den Schaft in einer gewindefreien Durchgangsbohrung durchsetzt, - wobei sie dann als, vorzugsweise selbstschneidende, Knochenschraube in den Calcarbogen eingeschraubt ist - wird der Knochen gegen das Implantat gezogen und so bei der Implantation sofort eine Primärfixation des Schaftes im proximalen Bereich sichergestellt; darüberhinaus sind dadurch der Schaft und der Knochen in diesem Bereich fest miteinander verbunden, so dass keine Kraftpulsationen mehr auf den Knochen einwirken können.

Als Erleichterung für ein richtiges, d.h. auf die Bohrung im Schaft passendes "Setzen" der im Knochen notwendigen "Kanäle" hat sich bewährt, wenn die Bohrung mindestens annnähernd senkrecht zur Prothesenhalsachse verläuft.

Im folgenden wird die Erfindung anhand eines Ausführungsbeispiels näher erläutert.

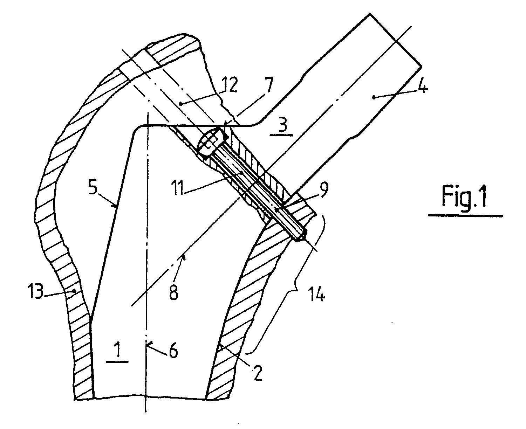

Die einzige Figur zeigt schematisch den proximalen Bereich eines erfindungsgemäss ausgebildeten Schaftes für eine Femurkopfprothese, eingesetzt in einen im Längsschnitt dargestellten Femurknochen.

Der nur in seinem proximalen Teil dargestellte Schaft 1, dessen Querschnitt blattartig, d.h. in der Form eines relativ schmalen Rechteckes, ausgebildet sein oder eine beliebige andere Querschnittform - wie beispielsweise quaderförmig, prismatisch oder abgerundet - aufweisen kann, verläuft medial in einem Bogen 2 zum Prothesenhals 3, in den er kragenlos übergeht; die Achse des Prothesenhales 3 ist mit 8 bezeichnet. Auf dem Prothesenhals 3 sitzt ein konischer Zapfen 4, auf den ein nicht gezeigter, kugelförmiger Gelenk- oder Prothesenkopf aufgesetzt wird.

Die laterale Begrenzung des Schaftes 8 verbreitert sich zunächst von distal nach proximal konisch, ehe sie in eine zur Längsmittelachse 6 hin verlaufende Schrägfläche übergeht; diese endet in einer horizontalen Schulter 7, die den Uebergang zum Prothesenhals 3 bildet.

Mindestens nahezu normal zur Oberfläche des medialen Bogens 2 und ebenfalls senkrecht zur Prothesenhalsachse 8 erstreckt sich eine Bohrung 9 durch den Schaft 1; sie ist als Durchgangsbohrung ausgebildet.

Die Bohrung 9 nimmt eine Schraube 11 auf, die beim Ausführungsbeispiel eine selbstschneidende Knochenschraube ist; sie verläuft durch einen operativ vorgebohrten Kanal 12 im Knochen 13 hindurch von lateral/proximal schräg nach medial/distal und schraubt sich in den Calcarbogen 14 des Knochens 13 selbstschneidend ein.

Das "Setzen" des Kanals 12 am richtigen Ort ermöglicht beispielsweise eine nicht gezeigte Bohrschablone, die auf den Zapfen 4 passgenau aufgesetzt wird. Neben dem Abstand zwischen dem Ende des Zapfens 4 und der Bohrung 9 längs der Prothesenhalsachse 8 werden damit die für ein Fluchten des Kanals 12 mit der Bohrung 9 notwendige "richtige Steigung" des Kanals 12 relativ zur Prothesenhalsachse 8 und - mit Hilfe einer seitlich an den Prothesenhals 3 anliegenden Abstützung - die Lage des Kanals 12 in Richtung anterior/posterior festgelegt, ehe der Kanal 12 im Knochen 13 geschaffen wird.

**Ansprüche**

1. Kragenloser Schaft (1) für eine Femurkopfprothese, der medial in einem Bogen (2) in den Prothesenhals (3) übergeht und im eingesetzten Zustand in seinem distalen Bereich fest im Knochen (13) verkeilt ist, wobei im proximalen Bereich (14) des medialen Bogens (2) für die Aufnahme einer Schraube (11) eine Bohrung (9) vorgesehen ist, die den Schaft (1) als Durchgangsbohrung durchsetzt und mindestens annähernd normal durch die Oberfläche des medialen Bogens (2) verläuft, sowie eine Auflagefläche für den Kopf einer Knochenschraube (11) enthält, dadurch gekennzeichnet, dass die Bohrung (9) für die Aufnahme der Knochenschraube (11) gewindefrei ausgebildet ist.

2. Kragenloser Schaft nach Anspruch 1, dadurch gekennzeichnet, dass die Bohrung (9) mindestens annähernd senkrecht zur Prothesenhalsachse (8) verläuft.

**Claims**

1. A collarless stem (1) for a femoral head prosthesis, the stem merging medially in a curve (2) into the prosthesis neck (3) and being wedged tightly in the bone (13) in its distal zone in the state at which introduced, the proximal zone (14) of the medial curve (2) being formed with a bore (9) to receive a screw (11), the bore (9) being a continuous bore through the stem (1) and extending through the surface of the medial bend (2) at least substantially perpendicularly to such surface and containing a bearing surface for the head of a bone screw (11), characterised in that the bore (9) for receiving the bone screw (11) is devoid of screwthreading.

2. A collarless stem according to claim 1, characterised in that the bore (9) extends at least substantially perpendicularly to the axis (8) of the prosthesis neck.

**Revendications**

1. Tige (1) sans collerette pour une prothèse de tête fémorale dont la transition avec le col prothétique (3) du côté interne s'effectue en décrivant un arc (2), et solidement coincée en position dans l'os (l3) par sa région distale, la zone proximale (14) de l'arc interne (2) étant percée d'un trou (9) destiné à recevoir une vis (11), qui traverse la tige (1) de part en part, s'étend au moins à peu près perpendiculairement à la surface de l'arc interne (2), et présente une surface d'appui pour la tête d'une vis (11) insérable dans l'os, caractérisée par le fait que le trou (9), conçu pour recevoir la vis (11) insérable dans l'os, est réalisé sans filetage.

2. Tige sans collerette selon la revendication 1, caractérisée par le fait que le trou (9) s'étend au moins à peu près perpendiculairement à l'axe (8) du col prothétique.

Fig.1